# EUROPEAN PATENT SPECIFICATION

(11) **EP 3 328 366 B1**
(45) Date of publication and mention of the grant of the patent: **14.04.2021**
(21) Application number: 16754550.8
(22) Date of filing: 19.07.2016
(51) Int. Cl.: A61K 31/095, A61K 38/00, A61K 31/185, A61K 31/19, A61K 31/295, A61K 33/26, A61P 33/00, A61K 9/00

(54) **AQUEOUS FORMULATION COMPRISING SDS, TCA AND A COLLOIDAL SUSPENSION OF IRON (III) FOR USE IN THE TOPICAL TREATMENT OF MICRO-ORGANISMS**
WÄSSRIGE FORMULIERUNG MIT SDS, TCA UND EINER KOLLOIDALEN SUSPENSION AUS EISEN (III) ZUR VERWENDUNG IN DER TOPISCHEN BEHANDLUNG VON MIKROORGANISMEN
FORMULATION AQUEUSE COMPRENANT SDS, TCA ET UNE SUSPENSION COLLOÏDALE DE FER (III) POUR UTILISATION DANS LE TRAITEMENT TOPIQUE DE MICRO-ORGANISMES

(30) Priority: 29.07.2015 IT UB20152612
(43) Date of publication of application: 06.06.2018
(73) Proprietor: Aint S.r.l., 30135 Venezia (IT)
(72) Inventor: MAGRO, Massimiliano, I-30135 Venezia (IT); VIANELLO, Fabio, I-30135 Venezia (IT); BRAGA, Mauro, I-30135 Venezia (IT)
(74) Representative: Hoffmann Eitle
(86) International application number: PCT/IB2016/054295
(87) International publication number: WO 2017/017561

(56) References cited:
- WO-A1-2009/011228
- KR-A- 20000 018 206
- LESIN V I ET AL: "Colloidal catalysts based on iron(III) oxides. 1. Decomposition of hydrogen peroxide", COLLOID JOURNAL, KLUWER ACADEMIC PUBLISHERS-PLENUM PUBLISHERS, NE, vol. 74, no. 1, 4 February 2012 (2012-02-04), pages 85-90, XP035012093, ISSN: 1608-3067, DOI: 10.1134/S1061933X12010103
- KASAIKINA O.T ET AL.: "Colloidal cytalysts on the base of iron(3+) oxides for oxidative treatmetn of biomass", CATAL. SUSTAIN. ENERGY, vol. 2, 1 January 2014 (2014-01-01), pages 21-27, XP002755872,
- EGINTON P J ET AL: "Changes in the strength of attachment of micro-organisms to surfaces following treatment with disinfectants and cleansing agents.", LETTERS IN APPLIED MICROBIOLOGY AUG 1998, vol. 27, no. 2, August 1998 (1998-08), pages 101-105, XP002755873, ISSN: 0266-8254

## Description

This invention relates to a formulation for the treatment of animals, preferably mammals, more preferably of horses, cattle, buffalo or sheep, and a process for the manufacture of a formulation for the treatment of animals, preferably mammals.

It is known that small, medium and large pets, farm animals or food-producing animals, for example horses or cattle, can periodically suffer from skin or hoof diseases of various kinds, which may have slow or difficult courses and that, in the worst situations, can lead to extremely serious health consequences for the animal.

These diseases are therefore a risk to the well-being and health of the animals (from pets to race horses), as well as obvious disadvantages for their sale. It is therefore not surprising that there are currently numerous compositions on the market for the care of the skin and hooves of animals.

However, there is a growing concern about some of these compositions, as their components and any metabolites, can be toxic in varying degrees to the animal affected by the disease: some products are known or suspected carcinogens, others are toxic or generate components that can be harmful

KR20000018206 discloses a skin cleaning composition with wound recovery properties comprising trichloroacetic acid and sodium lauryl sulfate.

The sale of some of the known compositions is actually already banned in many countries, while in other territories their sale will be prohibited in the near future.

Merely by way of example, many compositions contain formalin (aqueous formaldehyde solution), currently prohibited, or antibiotics - such as tetracyclines - which require costly certification in relation to the exposure of animals to such agents.

Consequently, there is a strong need to find alternatives to those currently available substances that do not have the above-mentioned drawbacks.

This invention is placed in the preceding context, proposing to provide a formulation, advantageously of the topical type, designed to act in a localised manner and able to act as a valid alternative to the currently known compositions.

This purpose is achieved by means of a formulation for use in the topical treatment of diseases caused by pathogen micro-organisms in animals, preferably mammals, comprising or consisting of the following components:
i) sodium dodecyl sulphate (SDS);
ii) 2, 2, 2-trichloroacetic acid (TCAA) ;
iii) a stable colloidal suspension of (nano-)particles of iron (III), advantageously as a catalyst to generate in water, and *in vivo,* cytotoxic substances known as reactive oxygen species.

The formulation of the invention can advantageously be used with success in the treatment of a plurality of diseases that affect the skin and the hooves of animals, more precisely diseases that include cutaneous mycoses or diseases of the hoof of horses, cattle, buffalo or sheep.

Among these equine, bovine or buffalo cutaneous mycoses or among hoof diseases, we mention, purely by way of non-limiting example: onychomycosis, white line disease, equine coat dermatitis, dermatitis of the bovine hoof, digital dermatitis, interdigital dermatitis, bovine dermatophytosis, breast lesions, particularly along the suspensory ligament of the breast or in the contact area between the breast and the leg of the animal, preferably of the mammal.

With regard to component i) of the formulation, SDS exerts a perturbing action on the cell membranes of bacteria, and denatures the proteins weakening intramolecular interactions. In addition, also being a detergent, SDS allows the dispersion in aqueous media of hydrophobic compounds insoluble in water, including the proteins of the cell membranes, which are thus extracted and solubilised. Finally, this detergent reduces the surface tension of the formulation, in this way allowing a greater permeation through the pores of animal tissues, and promoting the contact of the formulation with any hidden micro-organisms.

As regards component ii), the use of TCAA implies a molecular mechanism that is currently not clarified and does not allow defining with certainty the modalities through which the precipitation of the proteins occurs.

Various studies have suggested that the acidic nature of TCAA is important for the induction of conformational changes that trigger the precipitation of the proteins. This notwithstanding, it has been shown that the acidic nature of TCAA does not contribute in an exclusive manner to the related precipitation activity of the proteins. Further studies have also suggested that TCAA acts as a sequestrant of the water bound to the proteins, thus forcing the latter to come out of the solution and precipitate.

Finally, as regards component iii), it has been found that, in the stressful conditions induced by SDS and TCAA, this catalyst promotes a localised and *in vivo* production of cytotoxic substances known as reactive oxygen species.

In other words, component iii) preferably acts as a pro-oxidant, capable of inducing oxidative stress through the creation of reactive oxygen species, such stress being suitable to cause damage to cells or tissues of pathogenic micro-organisms.

It is a non-trivial technical effect since it is appropriate to point out that, in normal and neutral pH conditions, this component does not exhibit any cytotoxic effect on tissues. Conversely, in the formulation of the invention, this component actively participates in the Fenton and Haber-Weiss reactions producing highly reactive radical species, and leading to the elimination of fungus or bacterial cells from the surface of the skin or hoof.

Since the cell walls of the micro-organisms can be extremely strong, and by virtue of the fact that the skin of the animals' hooves normally presents wrinkles and pores, it becomes a natural obstacle to the elimination of biological contaminants that may be present.

Therefore, in order to obtain a localised cytotoxic action without the use of substances that are toxic, carcinogenic or hazardous for the animal, this invention exploits the combined action of the above-mentioned components i)-iii). In fact, these components were selected so as to exert a synergistic action to carry out the discussed biocidic action.

Advantageously, the previously described formulation proved to be an excellent skin healing agent.

Moreover, the above formulation is advantageously odourless, and does not generate stains of any kind on the skin.

Preferably, the (nano-)particles of iron (III) usable in this invention are manufactured according to the procedure discussed in document WO2012010200A1.

Still more preferably, the (nano-) particles in the invention are able to create stable colloidal suspensions in aqueous phase for at least six months, without the need for a coating, by means of treatment with ultrasonic bath.

With regard to the amounts of components i)-iii), various embodiments - implementable independently from each other - could provide that:
- component i) is comprised in a percentage by weight of about 0.05-5%wt compared to the total weight of the formulation;
- component ii) is comprised in a percentage by weight of about 8-15%wt compared to the total weight of the formulation; and/or
- component iii) is comprised in a percentage by weight of about 0.1-3%wt, preferably about 0.5-1.5%wt, compared to the total weight of the formulation.

The said objective is also achieved by a process of obtaining a formulation for use in the topical treatment of diseases caused by pathogen micro-organisms in animals, preferably mammals, comprising one or more steps of mixing sodium dodecyl sulphate, 2,2,2-trichloroacetic acid and a stable colloidal suspension of (nano-)particles of iron (III) in water, for example at ambient temperature, to obtain said formulation.

This process has proved particularly suitable for preparing the above described formulation. Therefore, even where this is not expressly stated, this process comprises all the characteristics (for example quantitative) preferred or advantageous deducible from the preceding description.

This description also covers the use of any of the following components:
i) sodium dodecyl sulphate;
ii) 2,2,2-trichloroacetic acid; or
iii) a stable colloidal suspension of (nano-)particles of iron (III);
   for the cure of skin afflictions of animals, preferably mammals, such as horses, cattle, buffalo or sheep.

Innovatively, the components of the formulation of this invention allow exercising a synergistic action in order to eliminate pathogenic micro-organisms from the skin of animals, preferably mammals, in a manner extremely localised and non-toxic to the animal itself.

Advantageously, the increased contact of this formulation with any hidden micro-organisms is a fundamental prerequisite for a real applicability for the treatment of specific diseases in animals, for example onychomycosis.

Advantageously, in certain subjects, the above formulation was sufficient for the substantial complete resolution of various pathologies.

Advantageously, the formulation of this invention does not need special equipment for its preparation.

Advantageously, the components described above act together in a synergistic way, so as to increase the curative effect of the formulation.

Advantageously, the formulation of this invention has an effective healing action on animals, in order to repair the underlying lesion.

## Claims

1. Aqueous formulation for use in the topical treatment of diseases caused by pathogen micro-organisms in animals, preferably mammals, comprising or consisting of the following components:
i) sodium dodecyl sulphate;
ii) 2,2,2-trichloroacetic acid;
iii) a stable colloidal suspension of (nano-)particles of iron (III) as a catalyst to generate in water, and *in vivo,* cytotoxic substances known as reactive oxygen species.

2. Formulation for use in the topical treatment of diseases caused by pathogen micro-organisms in animals according to the previous claim, wherein component i) is comprised in a percentage by weight of about 0.05-5%wt compared to the total weight of the formulation.

3. Formulation for use in the topical treatment of diseases caused by pathogen micro-organisms in animals according to any of the previous claims, wherein component ii) is comprised in a percentage by weight of about 8-15%wt compared to the total weight of the formulation.

4. Formulation for use in the topical treatment of diseases caused by pathogen micro-organisms in animals according to any of the previous claims, wherein component iii) is comprised in a percentage by weight of about 0.1-3%wt compared to the total weight of the formulation.

5. Formulation for use in the topical treatment of diseases caused by pathogen micro-organisms in animals according to any of the previous claims, wherein said diseases comprise cutaneous mycoses, or hoof diseases of equines, bovines, ovines or buffalo.

6. Formulation for use in the topical treatment of diseases caused by pathogen micro-organisms in animals according to the previous claim, wherein said equine, bovine, ovine or buffalo cutaneous mycoses or said hoof diseases are selected from the group consisting of: onychomycosis, white line disease, equine coat dermatitis, dermatitis of the bovine hoof, digital dermatitis, interdigital dermatitis, bovine dermatophytosis, breast lesions, particularly along the suspensory ligament of the breast or in the contact area between the breast and the leg of the bovine/equine.

7. Method of obtaining a formulation for use in the topical treatment of diseases caused by pathogen micro-organisms in animals, preferably mammals, comprising one or more steps of mixing sodium dodecyl sulphate, 2,2,2-trichloroacetic acid and a stable colloidal suspension of (nano-)particles of iron (III) in water, for example at ambient temperature, to obtain said formulation.

## Patentansprüche

1. Wässrige Formulierung zur Verwendung bei der topischen Behandlung von Erkrankungen, die durch pathogene Mikroorganismen in Tieren, vorzugsweisen Säugetieren, verursacht werden, umfassend oder bestehend aus den folgenden Bestandteilen:
i) Natriumdodecylsulfat;
ii) 2,2,2-Trichloressigsäure
iii) eine stabile kolloidale Suspension aus Eisen(III)-(Nano-)partikeln als ein Katalysator, um in Wasser und *in vivo* zytotoxische Substanzen zu erzeugen, die als reaktive Sauerstoffspezies bekannt sind.

2. Formulierung zur Verwendung bei der topischen Behandlung von Erkrankungen, die durch pathogene Mikroorganismen in Tieren verursacht werden gemäß dem vorhergehenden Anspruch, wobei der Bestandteil i) in einem Gewichtsprozentsatz von etwa 0,05-5 Gew.-%, bezogen auf das Gesamtgewicht der Formulierung, umfasst ist.

3. Formulierung zur Verwendung bei der topischen Behandlung von Erkrankungen, die durch pathogene Mikroorganismen in Tieren verursacht werden gemäß einem der vorhergehenden Ansprüche, wobei der Bestandteil ii) in einem Gewichtsprozentsatz von etwa 8-15 Gew.-%, bezogen auf das Gesamtgewicht der Formulierung, umfasst ist.

4. Formulierung zur Verwendung bei der topischen Behandlung von Erkrankungen, die durch pathogene Mikroorganismen in Tieren verursacht werden, gemäß einem der vorhergehenden Ansprüche, wobei der Bestandteil iii) in einem Gewichtsprozentsatz von etwa 0,1-3 Gew.-%, bezogen auf das Gesamtgewicht der Formulierung, umfasst ist.

5. Formulierung zu Verwendung bei der topischen Behandlung von Erkrankungen, die durch pathogene Mikroorganismen in Tieren verursacht werden gemäß einem der vorhergehenden Ansprüche, wobei die Erkrankungen kutane Mykosen oder Huferkrankungen bei Pferden, Rindern, Schafen oder Büffeln umfassen.

6. Formulierung zur Verwendung bei der topischen Behandlung von Erkrankungen, die durch pathogene Mikroorganismen in Tieren verursacht werden, gemäß dem der vorhergehenden Anspruch, wobei die kutanen Mykosen oder Huferkrankungen bei Pferden, Rindern, Schafen oder Büffeln ausgewählt sind aus der Gruppe, bestehend aus: Onychomykose, Weißlinienkrankheit, atopische Dermatitis beim Pferd, Dermatitis des Rinderhufes, *Dermatitis digitalis, Dermatitis interdigitalis,* Rinderdermatophytose, Brustläsionen, insbesondere entlang des Suspensoriumsbandes der Brust oder im Kontaktbereich zwischen der Brust und dem Bein des Rindes/Pferdes.

7. Verfahren zur Erhaltung einer Formulierung zur Verwendung in der topischen Behandlung von Erkrankungen, die durch pathogene Mikroorganismen bei Tieren, vorzugsweisen Säugetieren, verursacht werden, umfassend einen oder mehrere Schritte des Mischens von Natriumdodecylsulfat, 2,2,2-Trichloressigsäure und einer stabilen kolloidalen Suspension aus Eisen(III)-(Nano-) partikeln in Wasser, zum Beispiel bei Raumtemperatur, um die Formulierung zu erhalten.

## Revendications

1. Formulation aqueuse pour une utilisation dans le traitement topique de maladies dues à des microorganismes pathogènes chez les animaux, de préférence mammifères, comprenant ou consistant en les composants suivants ;
i) du dodécylsulfate de sodium ;
ii) de l'acide 2,2,2-trichloroacétique ;
iii) une suspension colloïdale stable de (nano)particules de fer(III) en tant que catalyseur pour générer dans l'eau, et *in vivo,* des substances cytotoxiques connues en tant qu'espèces réactives de l'oxygène.

2. Formulation pour une utilisation dans le traitement topique de maladies dues à des microorganismes pathogènes chez les animaux selon la revendication précédente, dans laquelle le composant i) est présent en un pourcentage en poids d'environ 0,05 à 5 % en poids par rapport au poids total de la formulation.

3. Formulation pour une utilisation dans le traitement topique de maladies dues à des microorganismes pathogènes chez les animaux selon l'une quelconque des revendications précédentes, dans laquelle le composant ii) est présent en un pourcentage en poids d'environ 8 à 15 % en poids par rapport au poids total de la formulation.

4. Formulation pour une utilisation dans le traitement topique de maladies dues à des microorganismes pathogènes chez les animaux selon l'une quelconque des revendications précédentes, dans laquelle le composant iii) est présent en un pourcentage en poids d'environ 0,1 à 3 % en poids par rapport au poids total de la formulation.

5. Formulation pour une utilisation dans le traitement topique de maladies dues à des microorganismes pathogènes chez les animaux selon l'une quelconque des revendications précédentes, dans laquelle lesdites maladies comprennent les mycoses cutanées, ou les maladies des sabots des équidés, bovidés, ovidés ou buffles.

6. Formulation pour une utilisation dans le traitement topique de maladies dues à des microorganismes pathogènes chez les animaux selon la revendication précédente, dans laquelle lesdites mycoses cutanées ou lesdites maladies des sabots des équidés, bovidés, ovidés ou buffles sont choisies dans le groupe constitué par : l'onychomycose, la maladie de la ligne blanche, la dermatite de la robe des chevaux, la dermatite des sabots des bovins, la dermatite digitale, la dermatite interdigitale, la dermatophytose des bovins, les lésions du poitrail, en particulier le long du ligament suspenseur du poitrail ou dans la zone de contact entre le poitrail et le membre du bovidé/équidé.

7. Procédé d'obtention d'une formulation pour une utilisation dans le traitement topique de maladies dues à des microorganismes pathogènes chez les animaux, de préférence mammifères, comprenant une ou plusieurs étapes de mélange de dodécylsulfate de sodium, d'acide 2,2,2-trichloroacétique et d'une suspension colloïdale stable de (nano)particules de fer(III) dans de l'eau, par exemple à la température ambiante, pour que soit obtenue ladite formulation.
